# EUROPEAN PATENT APPLICATION

(11) **EP 4 091 621 A1**
(43) Date of publication of application: **23.11.2022**
(21) Application number: 21741423.4
(22) Date of filing: 18.01.2021
(51) Int. Cl.: A61K 38/00, A61P 25/00

(54) **METHOD FOR TREATMENT OF NERVE INJURY AND RELATED DISEASE**

(30) Priority: 17.01.2020 WO PCT/CN2020/072747
(71) Applicant: Talengen International Limited, Hong Kong 999077 (HK)
(72) Inventor: LI, Jinan, Shenzhen City, Guangdong 518020 (CN)
(74) Representative: karo IP
(86) International application number: PCT/CN2021/072505
(87) International publication number: WO 2021/143906

(57) **Abstract**

Provided is a method for treatment of nerve injury and a related disease, comprising: administrating a therapeutically effective amount of a component of plasminogen activation pathway to a subject. Also provided are a medicament, a pharmaceutical composition, a product, and a kit which comprise a component of plasminogen activation pathway for treating the above diseases.

## Description

### TECHNICAL FIELD

The present application relates to a method for treating nerve injury and related diseases, such as paralysis, comprising: administrating to a subject an effective amount of a component of plasminogen activation pathway or a compound related thereto, such as plasminogen, to repair damaged nerves and improve clinical symptoms and signs.

### BACKGROUND ART

The nervous system includes the central nervous system (brain, spinal cord) and the peripheral nervous system (peripheral nervous tissue). Nervous system damage is caused by a variety of factors: (1) physical damage, which directly causes damage to nerve tissue at the site of injury, such as tissue damage of brain nerve or spinal cord injury caused by trauma; (2) temporary or permanent ischemia or hypoxia of part of the nervous system, such as tissue damage of brain nerve caused by stroke or cerebral embolism; (3) exposure to neurotoxins, such as chemicals used to treat cancer, etc.; (4) chronic metabolic diseases, such as peripheral nerve injury caused by diabetes or renal dysfunction, etc.

Spinal cord nerve injury is a general term for pathological conditions in which trauma or disease leads to the destruction or impairment of spinal cord nerve structure and function. For example, spinal cord trauma, spinal compression fracture, transverse myelitis, spinal cord tumor, vertebral tuberculosis, and syringomyelia may lead to spinal cord injury, thereby resulting in paralysis. The paraplegia caused by transverse lesions above cervical enlargement is high paraplegia, and the paraplegia caused by spinal cord injury below the third thoracic vertebra is paraplegia of both lower limbs. In the acute stage of spinal cord injury, the loss of sensation, movement, and reflex of the bilateral limbs below the injured level, as well as the loss of bladder and anal sphincter functions results in spinal cord shock. There is currently no ideal treatment for nerve injury, including spinal cord injury.

### SUMMARY OF THE APPLICATION

The research of the present application found that plasminogen may obviously promote the repair of nerve injury, promote the regeneration of myelin sheath of the injured nerve, and improve the related symptoms.

Particularly, the present application relates to the following:
1. A method for treating nerve injury, comprising: administrating to a subject a therapeutically effective amount of one or more compounds selected from the group consisting of: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.
2. The method according to claim 1, wherein the component of plasminogen activation pathway is selected from the group consisting of: plasminogen, recombinant human plasmin, Lys-plasminogen, Glu-plasminogen, plasmin, a variant and analog of plasminogen and plasmin comprising one or more kringle domains and protease domain of plasminogen and plasmin, mini-plasminogen, mini-plasmin, micro-plasminogen, micro-plasmin, delta-plasminogen, delta-plasmin, an activator of plasminogen, tPA and uPA.
3. The method according to claim 1, wherein the antagonist of fibrinolysis inhibitor is an inhibitor (e.g., an antibody) of PAI-1, complement C1 inhibitor, α2 antiplasmin or α2 macroglobulin.
4. The method according to any one of claims 1-3, wherein the nerve injury comprises spinal cord nerve injury, especially compressive spinal cord nerve injury.
   In some embodiments, the nerve injury is central nerve injury (e.g., spinal nerve injury) or peripheral nerve injury.
5. The method according to claim 4, wherein the spinal cord nerve injury comprises a spinal cord nerve injury caused by spinal compression fracture, transverse myelitis, spinal cord tumor, vertebral tuberculosis, or syringomyelia.
6. The method according to any one of claims 1-5, comprising: treating a nerve injury-related disease.
7. The method according to claim 6, wherein the nerve injury-related disease includes paralysis.
8. The method according to claim 7, wherein the paralysis is high paraplegia or paraplegia of both lower limbs.
9. The method according to any one of claims 1-8, wherein the compound promotes the repair of damaged nerve.
10. The method according to any one of claims 1-9, wherein the compound promotes the regeneration of nerve myelin sheath.
11. The method according to any one of claims 1-10, wherein the compound promotes the expression of spinal neurofilament protein.
12. The method according to any one of claims 1-11, wherein the compound promotes the recovery of sensory nerve function.
13. The method according to claim 12, wherein the compound promotes the recovery of pain sense.
14. The method according to any one of claims 1-13, wherein the compound is plasminogen.
   In some embodiments, the present application relates to one or more activities or functions of plasminogen selected from the group consisting of: promoting the regeneration of nerve myelin sheath, promoting the expression of spinal cord neurofilament protein and the regeneration of nerve fiber, promoting the recovery of sensory nerve function or the recovery of motor nerve function, promoting the recovery of pain sense, promoting the expression of synaptophysin, and promoting the recovery of the Nissl body level of an injured nerve cell.
15. In some embodiments, the above plasminogen has at least 75%, 80%, 85%, 90%, 95%, 96%, 97%, 98% or 99% sequence identity with SEQ ID NO: 2 and still has plasminogen activity (e.g., proteolytic activity or lysine binding activity).
16. In some embodiments, the above plasminogen is a protein comprising an active fragment of plasminogen and still having plasminogen activity (e.g., proteolytic activity or lysine binding activity). In some embodiments, the active fragment of plasminogen has at least 75%, 80%, 85%, 90%, 95%, 96%, 97%, 98% or 99% sequence identity with the active fragment of plasminogen represented by SEQ ID NO: 14, or has 100% amino acid sequence identity with the active fragment of plasminogen represented by SEQ ID NO: 14.
17. The method according to any one of claims 1-14, wherein the plasminogen is selected from the group consisting of: Glu-plasminogen, Lys-plasminogen, mini-plasminogen, micro-plasminogen, delta-plasminogen or their variants retaining plasminogen activity (e.g., proteolytic activity or lysine binding activity).
18. The method according to any one of claims 1-14, wherein the plasminogen is natural or synthetic full-length human plasminogen, or a variant or fragment thereof still retaining plasminogen activity (e.g., proteolytic activity, or lysine binding active).

In any of the above embodiments of the application, the plasminogen may have at least 75%, 80%, 85%, 90%, 95%, 96%, 97%, 98% or 99% sequence identity with SEQ ID NO: 2, 6, 8, 10 or 12, and still have plasminogen activity (e.g., proteolytic activity or lysine binding activity). In some embodiments, the plasminogen is a protein with addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1 -3, 1-2, or 1 amino acid based on SEQ ID NO: 2, 6, 8, 10 or 12, and still has plasminogen activity (e.g., proteolytic activity or lysine binding activity). In some embodiments, the plasminogen comprises or has the amino acid sequence set forth in SEQ ID NO: 2, 6, 8, 10 or 12.

In some embodiments, the plasminogen is a protein comprising an active fragment of plasminogen and still having plasminogen activity, such as proteolytic activity, or lysine binding activity, or both proteolytic activity and lysine binding activity. In some embodiments, the plasminogen is selected from the group consisting of: Glu-plasminogen, Lys-plasminogen, mini-plasminogen, micro-plasminogen, delta-plasminogen, or variants thereof retaining plasminogen activity (e.g., proteolytic activity or lysine binding activity). In some embodiments, the plasminogen is natural or synthetic human plasminogen, or a variant or fragment thereof still retaining plasminogen activity (e.g., proteolytic activity or lysine-binding activity). In some embodiments, the plasminogen is a human plasminogen ortholog from a primate or rodent, or a variant or fragment thereof still retaining plasminogen activity. In some embodiments, the amino acid sequence of the plasminogen is represented by SEQ ID NO: 2, 6, 8, 10 or 12. In some embodiments, the plasminogen is human natural plasminogen.

In some embodiments, the subject is a human. In some embodiments, the subject is deficient or lacking in plasminogen. In some embodiments, the lack or deficiency of plasminogen is congenital, secondary and/or local.

In some embodiments, the pharmaceutical composition comprises a pharmaceutically acceptable carrier and plasminogen for use in the above methods. In some embodiments, the kit may be a prophylactic or therapeutic kit, comprising: (i) plasminogen for use in the above methods, and (ii) means for delivering the plasminogen to the subject. In some embodiments, the means is a syringe or vial. In some embodiments, the kit further comprises a label or instructions for instructing the administration of the plasminogen to the subject to perform any of the above methods.

In some embodiments, the product comprises: a container comprising a label; and further comprises (i) plasminogen or a pharmaceutical composition comprising the plasminogen for use in the above method, wherein the label instructs the administration of the plasminogen or composition to the subject to perform any of the above methods.

In some embodiments, the kit or product further comprises one or more additional means or containers containing other medicaments.

In some embodiments of the above methods, the plasminogen is administrated by systemic or topical administration for therapy, preferably by intravenous, intramuscular, subcutaneous, or intrathecal administration of plasminogen. In some embodiments of the above methods, the plasminogen is administrated in combination with a suitable polypeptide carrier or stabilizer. In some embodiments of the above methods, the plasminogen is administrated per day at the amount of 0.0001-2000 mg/kg, 0.001-800 mg/kg, 0.01-600 mg/kg, 0.1-400 mg/kg, 1-200 mg/kg, 1-100 mg/kg, or 10-100mg/kg (by per kilogram of body weight); or 0.0001-2000 mg/cm², 0.001-800 mg/cm², 0.01-600 mg/cm², 0.1-400 mg/cm², 1-200 mg/cm², 1-100 mg/cm², or 10-100 mg/cm² (by per square centimeter of body surface area), preferably repeating at least once, and preferably administrating at least daily.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

### BRIEF DESCRIPTION OF THE DRAWINGS

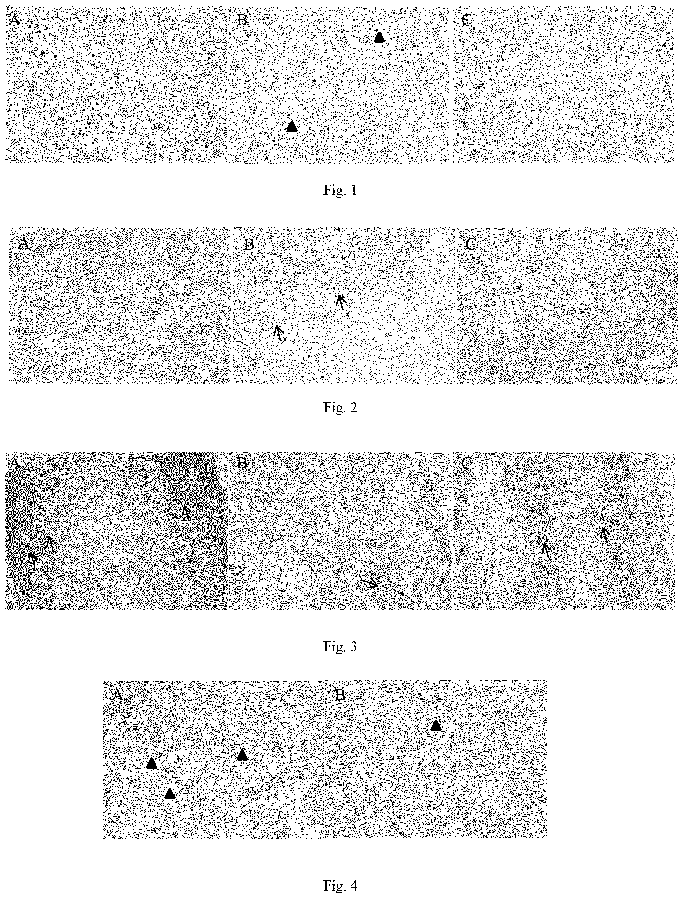

Figs. 1A-C show representative images of H&E staining of spinal cord after administrating plasminogen to the mice of spinal cord compression injury model for 7 days. A is sham operation group, B is the control group in which the mice are given the vehicle PBS (*hereinafter referred to as* vehicle PBS control group, or vehicle group), and C is the group in which the mice are given the plasminogen (*hereinafter referred to as* plasminogen group). The results show that the neurons and nerve fibers in sham operation group are in normal shape, and the cytoplasm is stained red; the junction parts of normal nerve and injured nerve from vehicle PBS control group and plasminogen group are respectively collected, a large area of damage voids (marked by triangles) may be seen in the junction parts from vehicle PBS control group, while the degree of nerve repair at the injured site in plasminogen group is significantly higher than that in vehicle PBS control group, and no obvious voids are found. It indicates that plasminogen may obviously promote the repair of injured spinal cord nerve in the mice of spinal cord compression injury model.
Figs. 2A-C show representative images of LFB staining of spinal cord after administrating plasminogen to the mice of spinal cord compression injury model for 7 days. A is sham operation group, B is vehicle PBS control group, and C is plasminogen group. The results show that the nerve myelin sheaths in the sham operation group (Fig. 2A) have complete and continuous structures; the nerve myelin sheaths in the vehicle PBS control group (Fig. 2B) show a larger area of disintegration (marked by arrows), the blue color is lighter, and the myelin sheaths are broken; compared with the vehicle PBS control group, the structures of myelin sheaths in the plasminogen group (Fig. 2C) are significantly more complete, and the blue color is deepened. This indicates that plasminogen may promote the regeneration of myelin sheath in the mice of spinal cord compression model.
Figs. 3A-C show representative images of the staining of spinal cord neurofilament protein (NFP) after administrating plasminogen to the mice of spinal cord compression injury model for 7 days. A is sham operation group, B is vehicle PBS control group, and C is plasminogen group. The results show that the expression of NFP (marked by arrows) in the plasminogen group is significantly higher than that in the vehicle PBS control group, and the expression of NFP is closer to that of the mice in the sham operation group. It indicates that plasminogen may promote the expression of NFP and promote the regeneration of spinal cord nerve fiber in the mice of spinal cord compression injury model.
Figs. 4A-B show representative images of H&E staining of spinal cord after administrating plasminogen to Plg-/- mice of spinal cord compression injury model for 7 days. A is vehicle PBS control group, and B is plasminogen group. The results show that the junction parts of normal nerves and injured nerves from the vehicle PBS control group and the plasminogen group are respectively collected. It can be seen that the normal structures of neurons and nerve fibers at the injury site are disappeared and replaced by repaired fibers, the cytoplasm is lightly stained, and inflammatory cells are infiltrated; however, in the vehicle PBS control group, large damage voids (marked by triangles) can be seen in the junction parts, while the degree of repair at the injured site in plasminogen group is significantly higher than that in vehicle PBS control group, and no obvious voids are found, and the infiltration of inflammatory cells is also significantly reduced. It indicates that plasminogen may obviously promote the repair of injured spinal cord in Plg-/- mice of spinal cord compression nerve injury model.

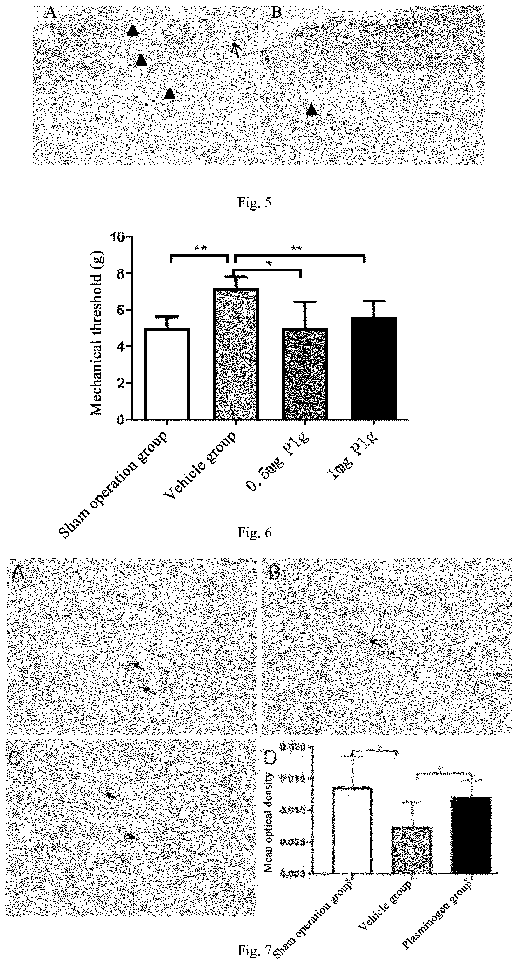

Figs. 5A-B show representative images of LFB staining of spinal cord after administrating plasminogen to the Plg-/- mice of spinal cord compression injury model for 7 days. A is vehicle PBS control group, and B is plasminogen group. The results show that the nerve myelin sheaths in the vehicle PBS control group display an obviously larger area of disintegration (marked by triangles), the blue color is lighter, and the myelin sheaths are broken (marked by arrows), by contrast, the sheath structures in the plasminogen group are obviously more complete and continuous, and the blue is darker. This indicates that plasminogen may significantly promote the repair of myelin damage in Plg-/- mice of the spinal cord compression nerve injury model.
Fig. 6 shows the detection results of threshold of Von-frey mechanical pain in C57 mice of spinal cord compression injury model after administrating plasminogen for 6 days. The results show that compared with the mice in the sham operation group, the mechanical threshold of the mice in the vehicle group is significantly increased, indicating that after establishment of the spinal cord compression model, the pain sensation of the mice is lost; the pain sense thresholds of the mice in plasminogen group A (1 mg) and plasminogen group B (0.5 mg) is significantly lower than that of the vehicle group, and the statistical difference is significant (^{∗} means P<0.05, ^{∗∗} means P<0.01). These results indicate that plasminogen may promote the recovery of pain sense in the mice of spinal cord compression model.
Figs. 7A-D show the results of NFP immunohistochemical staining of spinal cord in the mice of spinal cord transection model after administrating plasminogen for 28 days. A is sham operation group, B is vehicle PBS control group (also called the vehicle group, similarly hereinafter), C is plasminogen group, D is the quantitative analysis result of mean optical density. The results show that a certain level of NFP (marked by arrows) is expressed in the spinal cord of the mice in the sham operation group, and the expression of NFP in the spinal cord of molding part of the mice in the vehicle group is significantly lower than that of the mice in the sham operation group. The expression of NFP in the spinal cord of molding part of the mice in the plasminogen group is significantly higher than that of the vehicle group, and the statistical differences are significant (^{∗} means P<0.05). It indicates that plasminogen may promote the expression of NFP in spinal cord of the mice of spinal cord transection model.

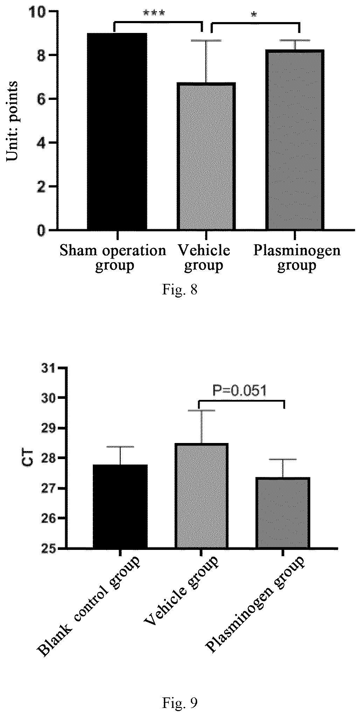

Fig. 8 show the results of BMS score of the mice of spinal cord transection model. The results show that the BMS score of the mice in the sham operation group is 9 points, the BMS score of the mice in the vehicle group is 6.75±1.92 points, which is significantly lower than that of the mice in the sham operation group; and the BMS score of the mice in the plasminogen group is 8.29±0.39 points, which is significantly higher than that of the mice in the vehicle group; and the statistical differences are significant (^{∗} means P<0.05, ^{∗∗∗} means P<0.001). It indicates that plasminogen may promote the recovery of motor function of the hindlimb in the mice of paraplegic model.
Fig. 9 shows RT-PCR detection results of the plasminogen mRNA of injured spinal cord in the mice of spinal cord transection model after administrating plasminogen for 6h. The results show that a certain level of plasminogen mRNA exists in the spinal cord of the mice in the blank control group, and the level of plasminogen mRNA in the spinal cord of the mice in the plasminogen group is significantly higher than that of the mice in the vehicle group, and the statistical difference is close to significant (P=0.051). It indicates that plasminogen may promote the transcription of plasminogen gene in spinal cord of the mice of paraplegic model.

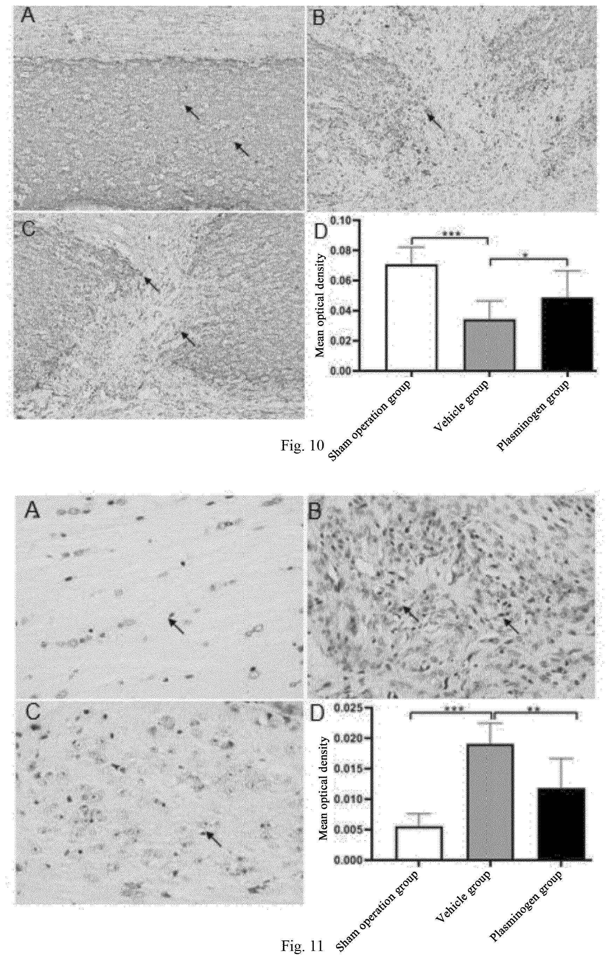

Figs. 10A-D show the results of immunohistochemical staining of synaptophysin in spinal cord in the mice of spinal cord transection model after administrating plasminogen for 28 days. A is sham operation group, B is vehicle group, C is plasminogen group, and D is the quantitative analysis result of mean optical density. The results show that a certain level of synaptophysin (marked by arrows) is expressed in spinal cord of the mice in the sham operation group, and the level of synaptophysin in the spinal cord of modeling part of the mice in the vehicle group is significantly lower than that of the mice in the sham operation group, and the statistical difference between the two groups is extremely significant (^{∗∗∗} means P<0.001). The level of synaptophysin in the spinal cord of the modeling part of the mice in the plasminogen group is significantly higher than that of the mice in the vehicle group, and the statistical difference is significant (^{∗} means P<0.05). It indicates that plasminogen may promote the expression of synaptophysin in the injured spinal cord of the mice of paraplegic model, and promote the repair of spinal cord injury.
Figs. 11A-D show the results of Nissl staining of spinal cord in the mice of spinal cord transection model after administrating plasminogen for 28 days. The results show that a certain level of Nissl bodies (marked by arrows) exists in the spinal cord of the mice in the sham operation group, and the level of Nissl bodies in the spinal cord of the modeling part of the mice in the vehicle group is significantly higher than that of the mice in the sham operation group, the statistical difference between the two groups is extremely significant (^{∗∗∗} means P<0.001). The level of Nissl bodies in the spinal cord of the modeling part of the mice in the plasminogen group is significantly lower than that in the vehicle group, and the statistical difference is extremely significant (^{∗∗} means P<0.01). It indicates that plasminogen may promote the recovery of the level of Nissl bodies in the injured spinal cord of the mice of paraplegic model, and promote the repair of spinal cord injury.

### DETAILED DESCRIPTION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by the liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complement, etc. Fibrinolysis inhibitors: including plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by the liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system in vivo: PAI-1, complement C1 inhibitor; α2 antiplasmin; α2 macroglobulin.

The term "component of plasminogen activation pathway" according to the present application encompasses:
1. plasminogen, Lys-plasminogen, Glu-plasminogen, micro-plasminogen, delta-plasminogen; variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and proteolytic domains.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the application encompasses a protein having at least 75%, 80%, 85%, 90%, 95%, 96%, 97%, 98% or 99% sequence identity with SEQ ID NO: 2, 6, 8, 10 or 12, and still retaining plasminogen activity (e.g., proteolytic activity, or lysine binding activity, or both proteolytic activity and lysine binding activity). For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity (e.g., proteolytic activity, or lysine binding activity, or both proteolytic activity and lysine binding activity). Particularly, the plasminogen variants according to the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen according to the present application may be a human plasminogen ortholog from a primate or rodent, or a variant thereof still retaining plasminogen activity (e.g., proteolytic activity, or lysine binding activity, or both proteolytic activity and lysine binding activity), for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle domains and proteolytic domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether the "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, it is measured by the level of activated plasmin activity based on enzymography, ELISA (enzyme-linked immunosorbent assay), and FACS (fluorescence-activated cell sorting method), for example, it is detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5): 1625-33; Gravanis I, Tsirka SE (February 2008). "Tissue-type plasminogen activator as a therapeutic target in stroke". Expert Opinion on Therapeutic Targets. 12(2): 159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining plasminogen activity (e.g., proteolytic activity, or lysine binding activity, or both proteolytic activity and lysine binding activity). In some embodiments, the plasminogen is natural or synthetic human plasminogen, or a conservative mutant variant or fragment thereof still retaining plasminogen activity. In some embodiments, the plasminogen is a human plasminogen ortholog from a primate or rodent or a conservative mutant variant or fragment thereof still retaining plasminogen activity. In some embodiments, the amino acid sequence of the plasminogen is represented by SEQ ID NO: 2, 6, 8, 10 or 12. In some embodiments, the plasminogen is a human natural plasminogen. In some embodiments, the plasminogen is a human natural plasminogen represented by SEQ ID NO: 2.

"A compound capable of directly activating plasminogen, or indirectly activating plasminogen by activating a upstream component of plasminogen activation pathway", refers to any compound capable of directly activating plasminogen, or indirectly activating plasminogen by activating a upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding and/or active domains of PAI-1, complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in the liver, and is abundantly present in the extracellular fluid. The content of plasminogen in plasma is approximately 2 µM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90kD, and it is a glycoprotein mainly synthesized in the liver and capable of circulating in the blood, the cDNA sequence encoding this amino acid sequence is represented by SEQ ID NO: 3. Full-length plasminogen contains seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle 1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is human natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a protease domain (PD)). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), its amino acid sequence is represented by SEQ ID NO: 10, and the cDNA sequence encoding this amino acid sequence is represented by SEQ ID NO: 9. Micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); in the present patent application, the sequence of micro-plasminogen refers to the patent document CN102154253A, the amino acid sequence is represented by SEQ ID NO: 12, and the cDNA sequence encoding this amino acid sequence is represented by SEQ ID NO: 11.

The structure of full-length plasminogen is also described in the article by Aisina et al. (Aisina RB, Mukhametova L I. Structure and function of plasminogen/plasmin system [J]. Russian Journal of Bioorganic Chemistry, 2014, 40(6):590-605). In this article, Aisina et al. describe that plasminogen comprises Kringle 1, 2, 3, 4, 5 domains and a serine protease domain (also called protease domain (PD)), wherein Kringles are responsible for binding of plasminogen to low or high molecular weight ligand, so that plasminogen transforms into a more open conformation that is more readily activated; the protease domain (PD) is residues Val562-Asn791; the Arg561-Val562 activating bond of plasminogen is specifically cleaved by tPA and UPA, thereby allowing plasminogen to change into plasmin.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

An "active fragment of plasminogen" refers to a fragment having the activity of binding to a lysine in the target sequence of a substrate (lysine-binding activity), or exerting a proteolytic function (proteolytic activity), or having a combination of proteolytic activity and lysine-binding activity. The technical solutions related to plasminogen according to the present application encompass the technical solutions of replacing plasminogen with an active fragment of plasminogen. In some embodiments, the active fragment of plasminogen according to the present application comprises or consists of a serine protease domain of plasminogen, preferably the active fragment of plasminogen according to the present application comprises or consists of SEQ ID NO: 14, or an amino acid sequence having at least 80%, 90%, 95%, 96%, 97%, 98%, 99% identity with SEQ ID NO: 14. In some embodiments, the active fragment of plasminogen according to the present application comprises or consists of one or more regions selected from the group consisting of: Kringle 1, Kringle 2, Kringle 3, Kringle 4, and Kringle 5. In some embodiments, the plasminogen according to the present application comprises a protein comprising the active fragment of plasminogen described above.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having not less than 60%, preferably not less than 75%, more preferably not less than 85%, or even most preferably not less than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:
Fraction X/Y times 100;
wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

As used herein, the terms "treatment" and "treating" refer to obtaining a desired pharmacological and/or physiological effect. The effect may be complete or partial prevention of the disease or symptoms thereof, and/or partial or complete cure of the disease and/or symptoms thereof; and includes: (a) preventing the occurrence of the disease in a subject, who may have predisposition of the disease, but is not yet diagnosed as having the disease; (b) inhibiting the disease, i.e., blocking its development; and (c) alleviating the disease and/or symptoms thereof, i.e., causing regression of the disease and/or symptoms thereof.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of plasminogen sufficient to prevent and/or treat a disease when administrated to a mammal or other subject for treating the disease. A "therapeutically effective amount" will vary depending on the plasminogen used, the severity of the disease and/or symptoms thereof in the subject to be treated, as well as the age, weight, and the like.

### Preparation of the Plasminogen According to the Present Application

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to operably link to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector typically replicates in a host organism as an episome or as an incorporated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a subject antibody-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., S. *cerevisiae*) and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization

In addition to microorganisms, mammalian cells (e.g., mammalian cells grown in in vitro cell culture) may also be used to express and produce the anti-Tau antibodies of the application (e.g., polynucleotides encoding the subject anti-Tau antibodies). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the subject antibody, and the like.

### Medicament Formulation

A therapeutic formulation may be prepared by mixing the plasminogen of desired purity with an optional pharmaceutical carrier, excipient, or stabilizer (Remington's Pharmaceutical Sciences, 16th edition, Osol, A. ed. (1980)), to form a lyophilized formulation or aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG). Preferred lyophilized anti-VEGF antibody formulation is described in WO 97/04801, which is incorporated herein by reference.

The formulations according to the present application may also contain not less than one active compound as required for the particular condition to be treated, preferably those compounds are complementary in activity and do not have side effects with each other. For example, antihypertensive medicaments, antiarrhythmic medicaments, medicaments for treating diabetes, etc.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen according to the present application for in vivo administration must be sterile. This may be easily achieved by filtration through sterilizing filters before or after lyophilization and reformulation.

The plasminogen according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot^{™} (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for not less than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

### Administration and Dosage

Administration of the pharmaceutical composition according to the present application may be accomplished by different means, e.g., intravenously, intraperitoneally, subcutaneously, intracranially, intrathecally, intraarterally (e.g., via the carotid artery), intramuscularly.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen according to the present application may be, for example, about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 1-10 mg/kg on consecutive days. Real-time evaluation of therapeutic efficacy and safety is required during the administration of the medicament of the present application.

### Product or Kit

One embodiment of the present application relates to a product or kit comprising the plasminogen or plasmin according to the present application for treating cardiovascular disease caused by diabetes and other related diseases. The product preferably comprises a container, and a label or package insert. Suitable containers are bottles, vials, syringes, etc. The container may be made of various materials such as glass or plastic. The container contains a composition which is effective for treatment of the disease or condition according to the present application and has a sterile access port (e.g., the container may be an intravenous solution pack or vial containing a stopper penetrable by a hypodermic needle). At least one active agent in the composition is plasminogen/plasmin. The label on or attached to the container indicates that the composition is used for treatment of cardiovascular disease caused by diabetes and related diseases according to the present application. The product may further comprise a second container containing a pharmaceutically acceptable buffer, such as phosphate buffered saline, Ringer's solution, and dextrose solution. It may further contain other materials required from a commercial and user standpoint, including other buffers, diluents, filters, needles and syringes. In addition, the product comprises a package insert with instructions for use, including, for example, instructing the user of the composition to administrate the plasminogen composition to the patient along with other medicaments for treatment of concomitant diseases.

### EXAMPLE

Human plasminogen used in the following examples is derived from plasma of a human donor, based on methods described in: Kenneth C Robbins, Louis Summaria, David Elwyn et al. Further Studies on the Purification and Characterization of Human Plasminogen and Plasmin. Journal of Biological Chemistry, 1965, 240(1): 541-550; Summaria L, Spitz F, Arzadon L et al. Isolation and characterization of the affinity chromatography forms of human Glu-and Lys-plasminogens and plasmins. J Biol Chem. 1976 Jun 25;251(12):3693-9; HAGAN JJ, ABLONDI FB, DE RENZO EC. Purification and biochemical properties of human plasminogen. J Biol Chem. 1960 Apr;235:1005-10, with process optimization, being purified from plasma of a human donor, with >98% human plasminogen monomer.

### Example 1: Plasminogen Promotes the Repair of Nerve Injury in the Mice of Spinal Cord Compression Injury Model

Fifteen 8-week-old C57 female mice are weighed and randomly divided into three groups, i.e., the sham operation group, the vehicle PBS control group, and the plasminogen group, with 5 mice in each group. All mice are anesthetized by intraperitoneal injection of sodium pentobarbital solution (50 mg/kg), incising the midline skin of the back, isolating the back T7-T9 muscles, and removing the T7-T9 vertebral plates. In the vehicle PBS control group and the plasminogen group, a microvascular clip (3.5×1.0 mm, Reward) is clamped at the T8 spinal cord for 1 min; in the sham operation group, only the vertebral plates are removed to avoid spinal dura mater injury. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously ^{[1]}. The administration is started 30 min after the operation, and the mice in the plasminogen group are given plasminogen by tail vein injection at 1 mg/0.1 ml/mouse/day, and the mice in the vehicle PBS control group is given the same volume of PBS in the same way, and the mice in the sham operation are not treated with administration. The first day of administration is day 1, and the administration cycle is 7 days. The mice are dissected on day 8 of administration, and the T7-T9 segments of spinal cord are taken out to fix in 4% paraformaldehyde. The fixed tissue samples are dehydrated with gradient ethanol and cleared with xylene before paraffin-embedding. The thickness of the spinal cord tissue cross-section is 4 µm. The sections are dewaxed and rehydrated, and then stained with hematoxylin and eosin (HE staining); after differentiation with 1% hydrochloric acid ethanol, ammonia water is used to return to blue, then dehydrating with gradient ethanol and sealing the sections. These sections are observed under a 200-fold optical microscope.

The results show that, the neurons and nerve fibers in sham operation group (Fig. 1A) are in normal shape, and the cytoplasm is stained red; the junction parts of normal nerve and injured nerve from vehicle PBS control group (Fig. 1B) and plasminogen group are respectively collected, a large area of damage voids (marked by triangles) may be seen in the junction parts from vehicle PBS control group, while the degree of nerve repair at the injured site in plasminogen group (Fig. 1C) is significantly higher than that in vehicle PBS control group, and no obvious voids are found. It indicates that plasminogen may obviously promote the repair of injured spinal cord nerve in the mice of spinal cord compression injury model.

### Example 2: Plasminogen Promotes the Regeneration of Nerve Myelin Sheath

Fifteen 8-week-old C57 female mice are weighed and randomly divided into three groups, i.e., the sham operation group, the vehicle PBS control group, and the plasminogen group, with 5 mice in each group. All mice are anesthetized by intraperitoneal injection of sodium pentobarbital solution (50 mg/kg), incising the midline skin of the back, isolating the back T7-T9 muscles, and removing the T7-T9 vertebral plates. In the vehicle PBS control group and the plasminogen group, a microvascular clip (3.5×1.0 mm, Reward) is clamped at the T8 spinal cord for 1 min; in the sham operation group, only the vertebral plates are removed to avoid spinal dura mater injury. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously ^{[1]}. The administration is started 30 min after the operation, and the mice in the plasminogen group are given plasminogen by tail vein injection at 1 mg/0.1 ml/mouse/day, and the mice in the vehicle PBS control group is given the same volume of PBS in the same way, and the mice in the sham operation are not treated with administration. The first day of administration is day 1, and the administration cycle is 7 days. The mice are dissected on day 8 of administration, and the T7-T9 segments of spinal cord are taken out to fix in 4% paraformaldehyde. The fixed tissue samples are dehydrated with gradient ethanol and cleared with xylene before paraffin-embedding. The thickness of the spinal cord tissue cross-section is 4 µm. After dewaxing to water, LFB staining is performed with myelin staining solution, then dehydrating with gradient ethanol, clearing with xylene, and sealing with neutral gum. The sections are observed under an optical microscope to take pictures.

LFB (luxol fast blue) staining is performed to stain myelin sheaths by fast blue method, and the degree of blue represents the amount of myelin sheaths. LFB staining is an effective method to study the localization of the corticospinal tract, and the morphological observation of myelin lesions, injury and regenerative repair ^{[2, 3]}_{∘}

The results show that the nerve myelin sheaths in the sham operation group (Fig. 2A) have complete and continuous structures; the nerve myelin sheaths in the vehicle PBS control group (Fig. 2B) show a larger area of disintegration (marked by arrows), the blue color is lighter, and the myelin sheaths are broken; compared with the vehicle PBS control group, the structures of myelin sheaths in the plasminogen group (Fig. 2C) are significantly more complete, and the blue color is deepened. This indicates that plasminogen may promote the regeneration of myelin sheath in the mice of spinal cord compression model.

### Example 3: Plasminogen Promotes the Expression of Spinal Neurofilament Protein

Fifteen 8-week-old C57 female mice are weighed and randomly divided into three groups, i.e., the sham operation group, the vehicle PBS control group, and the plasminogen group, with 5 mice in each group. All mice are anesthetized by intraperitoneal injection of sodium pentobarbital solution (50 mg/kg), incising the midline skin of the back, isolating the back T7-T9 muscles, and removing the T7-T9 vertebral plates. In the vehicle PBS control group and the plasminogen group, a microvascular clip (3.5×1.0 mm, Reward) is clamped at the T8 spinal cord for 1 min; in the sham operation group, only the vertebral plates are removed to avoid spinal dura mater injury. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously ^{[1]}. The administration is started 30 min after the operation, and the mice in the plasminogen group are given plasminogen by tail vein injection at 1 mg/0.1 ml/mouse/day, and the mice in the vehicle PBS control group is given the same volume of PBS in the same way, and the mice in the sham operation are not treated with administration. The first day of administration is recorded as day 1, and the administration cycle is 7 days. The mice are dissected on day 8 of administration, and the T7-T9 segments of spinal cord are taken out to fix in 4% paraformaldehyde. The fixed tissue samples are dehydrated with gradient ethanol and cleared with xylene before paraffin-embedding. The thickness of the tissue cross-section is 3 µm, after the sections are dewaxed and rehydrated, washing them once with water, then repairing with citric acid for 30 min, cooling at room temperature for 10 min, and rinsing gently with water; incubating with 3% hydrogen peroxide for 15 min, and circling the tissue with a PAP pen; blocking with 10% of goat serum (Vector laboratories, Inc., USA) for 1 hour, when the time is up, the goat serum is discarded. The tissue sections are incubated with rabbit-derived anti-neurofilament protein (NFP) antibody (Abeam, ab207176) at 4°C overnight, then washing twice with PBS, 5 min for each time; after incubating with goat anti-rabbit IgG (HRP) antibody (Abeam) secondary antibody at room temperature for 1 hour, washing twice with PBS, 5 min for each time. The color is developed with DAB kit (Vector laboratories, Inc., USA), after washing 3 times with water, counterstaining with hematoxylin for 30 seconds, returning to blue under running water for 5 min, and then washing once with PBS. After gradient dehydration, the tissue sections are cleared and sealed, then observed under a 200-fold optical microscope.

Neurofilament protein (NFP) is a protein that constitutes the intermediate filament of nerve cell axons. Its function is to provide elasticity to make nerve fibers stretch easily and prevent breakage, and it is of great significance in maintaining the cytoskeleton, stabilizing cell morphology and axonal transport ^{[4]}.

The results show that the expression of NFP (marked by arrows) in the plasminogen group (Fig. 3C) is significantly higher than that in the vehicle PBS control group (Fig. 3B), and the expression of NFP is closer to that of the mice in the sham operation group (Fig. 3A). It indicates that plasminogen may promote the expression of NFP and promote the regeneration of spinal cord nerve fiber in the mice of spinal cord compression injury model.

### Example 4: Plasminogen Promotes the Repair of Nerve Injury

Ten 8-week-old Plg-/- female mice are weighed and randomly divided into two groups, vehicle PBS control group and plasminogen group, with 5 mice in each group. All mice are anesthetized by intraperitoneal injection of sodium pentobarbital solution (50 mg/kg), incising the midline skin of the back, isolating the back T7-T9 muscles, and removing the T7-T9 vertebral plates. In the vehicle PBS control group and the plasminogen group, a microvascular clip (3.5×1.0 mm, Reward) is clamped at the T8 spinal cord for 1 min. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously ^{[1]}. The administration is started 30 min after the operation, and the mice in the plasminogen group are given plasminogen by tail vein injection at 1 mg/0.1 ml/mouse/day, and the mice in the vehicle PBS control group is given the same volume of PBS in the same way. The first day of administration is day 1, and the administration is continuously performed for 7 days. The mice are dissected on day 8 of administration, and the T7-T9 segments of spinal cord are taken out to fix in 4% paraformaldehyde. The fixed tissue samples are dehydrated with gradient ethanol and cleared with xylene before paraffin-embedding. The thickness of the spinal cord tissue cross-section is 4 µm. The sections are dewaxed and rehydrated, and then stained with hematoxylin and eosin (HE staining); after differentiation with 1% hydrochloric acid ethanol, ammonia water is used to return to blue, then dehydrating with gradient ethanol and sealing the sections. These sections are observed under a 200-fold optical microscope.

The results show that the neurons and nerve fibers in the sham operation group (Fig. 4A) are in normal shape, and the cytoplasm is stained red; the junction parts of normal nerves and injured nerves from the vehicle PBS control group (Fig. 4B) and the plasminogen group (Fig. 4C) are respectively collected, it can be seen that the normal structures of neurons and nerve fibers at the injury site are disappeared and replaced by repaired fibers, the cytoplasm is lightly stained, and inflammatory cells are infiltrated; however, in the vehicle PBS control group, large damage voids (marked by triangles) can be seen in the junction parts, while the degree of repair at the injured site in plasminogen group is significantly higher than that in vehicle PBS control group, and no obvious voids are found, and the infiltration of inflammatory cells is also significantly reduced. It indicates that plasminogen may obviously promote the repair of injured spinal cord in Plg-/- mice of spinal cord compression nerve injury model.

### Example 5: Plasminogen Promotes the Regeneration of Nerve Myelin Sheath in Plg-/- Mice of Spinal Cord Compression Injury Model

Ten 8-week-old Plg-/- female mice are weighed and randomly divided into two groups, vehicle PBS control group and plasminogen group, with 5 mice in each group. All mice are anesthetized by intraperitoneal injection of sodium pentobarbital solution (50 mg/kg), incising the midline skin of the back, isolating the back T7-T9 muscles, and removing the T7-T9 vertebral plates. In the vehicle PBS control group and the plasminogen group, a microvascular clip (3.5×1.0 mm, Reward) is clamped at the T8 spinal cord for 1 min. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously ^{[1]}. The administration is started 30 min after the operation, and the mice in the plasminogen group are given plasminogen by tail vein injection at 1 mg/0.1 ml/mouse/day, and the mice in the vehicle PBS control group is given the same volume of PBS in the same way. The first day of administration is day 1, and the administration is continuously performed for 7 days. The mice are dissected on day 8 of administration, and the T7-T9 segments of spinal cord are taken out to fix in 4% paraformaldehyde. The fixed tissue samples are dehydrated with gradient ethanol and cleared with xylene before paraffin-embedding. The thickness of the spinal cord tissue cross-section is 4 µm. After dewaxing to water, LFB staining is performed with myelin staining solution, then dehydrating with gradient ethanol, clearing with xylene, and sealing with neutral gum. The sections are observed under an optical microscope to take pictures.

The results show that the nerve myelin sheaths in the vehicle PBS control group (Fig. 5A) display an obviously larger area of disintegration (marked by triangles), the blue color is lighter, and the myelin sheaths are broken (marked by arrows), by contrast, the sheath structures in the plasminogen group (Fig. 5B) are obviously more complete and continuous, and the blue is darker. This indicates that plasminogen may significantly promote the repair of myelin damage in Plg-/- mice of the spinal cord compression nerve injury model.

### Example 6: Plasminogen Promotes the Recovery of Pain Sense in the Mice of Spinal Cord Compression Injury Model

Thirty-eight 7-week-old C57BL/6J female mice are selected and weighed one day before modeling, and are randomly divided into 2 groups according to the results of body weight; 7 mice in the sham operation group, and 31 mice in the model group. All mice are anesthetized by intraperitoneal injection of sodium pentobarbital solution (50 mg/kg), incising the midline skin of the back, isolating the back T5-T8 muscles, and removing the T6-T7 vertebral plates. In the model group, the microvascular clamp (30g, Kent Scientific Corporation) is clamped at the T6-T7 spinal cord for 1 min. The forceps should be inserted into the vertebral channel with the tip at the top of the vertebral plate to form an angle of 90° ^{[5]}; in the sham operation group only the vertebral plates are moved to avoid spinal dura mater injury. All mice are sutured immediately after the operation, intramuscularly injected with Tolfedine (0.1 mg/kg) and penicillin potassium (40,000 units/kg), and placed on a heating pad until they wake up. Infection is prevented during the operation, and the mice are manually assisted to urinate after the operation until they urinate spontaneously, and the observation is continued for 7 days. 7 days after the operation, all mice are subjected to scoring of body weight and BMS (scored blindly by two people) ^{[5]}. According to body weight and BMS scores, the mice in the model group are randomly divided into three groups: plasminogen group A, plasminogen group B, vehicle control group; and the beginning of administration is set as day 1 of administration, the mice in plasminogen group A are injected with plasminogen solution at 1 mg/100 µl/mouse through tail vein; the mice in plasminogen group B are injected at 0.5 mg/100 µl/mouse through tail vein; and the mice in the vehicle control group are injected with the vehicle solution at 100 µl/mice through tail vein, and the administration is continued for 6 days. On day 7, the Von-Frey mechanical tenderness test is performed on the left legs of the mice.

The results show that, compared with the mice in the sham operation group, the mechanical threshold of the mice in the vehicle group is significantly increased, indicating that after the establishment of the spinal cord compression injury model, the mice lost their pain sense; the pain sense threshold of the mice in plasminogen group A (1mg) and the pain sense threshold of the mice in plasminogen group B (0.5mg) are significantly lower than that of the mice in the vehicle group, and the statistical difference is significant (^{∗} means P<0.05, ^{∗∗} means P<0.01) (Fig. 6). These results indicate that plasminogen may promote the recovery of pain sense in the mice of spinal cord compression injury model.

### Example 7: Plasminogen Promotes the Expression of Spinal Neurofilament Protein in the Mice of Paraplegic Model

32 eight- to ten-week-old C57 female mice are weighed before modeling, and all mice are randomly divided into 2 groups according to body weight; 8 mice in the sham operation group, and 24 mice in the model group. Before the operation, the hairs on the back of the mice in the model group are removed under light anesthesia by Zoletil 50. After that, the mice are completely anesthetized by respiratory anesthesia, and placed in a prone position on an anesthetic gas machine turning on 2.5% isoflurane. The skin and muscles are opened surgically, and the T9 and T10 segments are exposed to perform the laminectomy. After the spinal cord is exposed, the right half of the spinal cord between T9 and T10 is transected along the coronal plane to cause a semi-transection injury, and the wound is sutured after completion of the injury. The mice in the sham operation group are anesthetized in the same way to perform laminectomy, and then the wound is directly sutured. After operation, all the mice are placed on a heating pad until the mice are awake up. Postoperative care includes daily injection of the analgesic Tolfedine and penicillin potassium (40,000 units/kg) for 4 days, ensuring that the mice may obtain food and water, and helping the mice to excrete urine until restoring the bladder's function of autonomous urination ^{[6]}. One week after the completion of the surgical modeling, the postoperative mice are subjected to open-field behavioral test and scoring. According to the testing results, the mice in the model group are randomly divided into two groups; the vehicle group and the plasminogen group, with 12 mice in each group. The mice in the sham operation group are not grouped. After the grouping is completed, the mice in the vehicle group and the sham operation group are injected with vehicle at 0.1 ml/mouse/day through tail vein, and the mice in the plasminogen group are injected with plasminogen at 1 mg/mouse/day through tail vein; the first day of administration is recorded as day 1, continuously administrating for 28 days. The mice are sacrificed on day 29, and the spinal cord of the modeling part is taken out to fix in 4% paraformaldehyde. The fixed tissue samples are dehydrated with gradient ethanol and cleared with xylene before paraffin-embedding. The thickness of the tissue cross-section is 3 µm, after the sections are dewaxed and rehydrated, washing them once with water, then repairing with citric acid for 30 min, cooling at room temperature for 10 min, and rinsing gently with water; incubating with 3% hydrogen peroxide for 15 min, and circling the tissue with a PAP pen; blocking with 10% of goat serum (Vector laboratories, Inc., USA) for 1 hour, when the time is up, the goat serum is discarded. The tissue sections are incubated with rabbit-derived anti-neurofilament protein (NFP) antibody (Abeam, ab207176) at 4°C overnight, then washing twice with PBS, 5 min for each time; after incubating with goat anti-rabbit IgG (HRP) antibody (Abeam) secondary antibody at room temperature for 1 hour, washing twice with PBS, 5 min for each time. The color is developed with DAB kit (Vector laboratories, Inc., USA), after washing 3 times with water, counterstaining with hematoxylin for 30 seconds, returning to blue under running water for 5 min, and then washing once with PBS. After gradient dehydration, the tissue sections are cleared and sealed, then observed under a 400-fold optical microscope.

The results show that a certain level of NFP (marked by arrows) is expressed in the spinal cord of the mice in the sham operation group (Fig. 7A), and the expression of NFP in the spinal cord of molding part of the mice in the vehicle group (Fig. 7B) is significantly lower than that of the mice in the sham operation group. The expression of NFP in the spinal cord of molding part of the mice in the plasminogen group (Fig. 7C) is significantly higher than that of the vehicle group, and the statistical differences are significant (^{∗} means P<0.05) (Fig. 7D). It indicates that plasminogen may promote the expression of NFP in spinal cord of the mice of spinal cord transection model.

### Example 8: Plasminogen Improves the Motor Function of Hindlimb in the Mice of Paraplegic Model

42 eight- to ten-week-old C57 female mice are weighed before modeling, and all mice are randomly divided into 2 groups according to body weight; 14 mice in the sham operation group, and 28 mice in the model group. The experimental mice are taken to perform pre-anesthesia, then the hairs on the back of the mice are removed. After that, the mice are completely anesthetized by respiratory anesthesia, and placed in a prone position on an anesthetic gas machine turning on 2.5% isoflurane. The skin and muscles are opened surgically, and the T9 and T10 segments are exposed to perform the laminectomy. After the spinal cord is exposed, the right half of the spinal cord between T9 and T10 is transected along the coronal plane to cause a semi-transection injury, and the wound is sutured after completion of the injury. After the mice in the sham operation group are anesthetized in the same way, the skin and muscles are surgically opened to expose the T9 and T10 segments, the wound is then sutured directly. The analgesic Tolfedine and penicillin potassium (200,000 units/kg; 80,000 units/ml) are administrated 24 and 72 hours before and after the operation. After the operation, all the mice are placed on a heating pad, and the neck should be kept horizontal, and the neck should not be bent until the mice are awake. Postoperative care: normally a mouse needs about 40-80ml/kg of liquid per day; after waking up from anesthesia, if the mice are found unable to eat or drink, they must be given normal saline by intraperitoneal injection to supplement body fluids, helping the mice to excrete urine until restoring the bladder's function of autonomous urination ^{[6]}. In order to prevent the wounds from being contaminated by feces, urine and padding material on the day after the operation, a clean tissue paper should be placed on the padding material in the PC box. Three days after the completion of the surgical modeling, the postoperative mice are subjected to open-field behavioral test and scoring. According to the testing results, 28 mice in the model group are randomly divided into two groups, the vehicle group and the plasminogen group, with 14 mice in each group; the mice in the sham operation group are not grouped. After the grouping is completed, the mice in the vehicle group and the sham operation group are injected with vehicle at 0.1 ml/mouse/day through tail vein, and the mice in the plasminogen group are injected with plasminogen at 1 mg/mouse/day through tail vein, the first day of administration is recorded as day 1, administrating continuously for 7 days. On day 8, the Basso Mouse Scale (BMS) test is performed according to the Table below.

The BMS scoring is specially used to evaluate the changes of hindlimb function in the mice with spinal cord injury, including primary and secondary scoring systems: the primary scoring is to observe the degree of joint motion of the hindlimb ankle, coordination, paw posture, trunk stability and tail posture of the mice. The secondary scoring is a supplementary modification of the primary scoring based on coordination, stability, etc. ^{[7]}.

| BMS (Basso Mouse Scale) Scoring | |
|---|---|
| 0 point | No joint motion |
| 1 point | Slight joint motion |
| 2 points | Great joint motion |
| 3 points | Touching the ground with sole of the paws or without weight support; or occasional, frequent, or constant motion on the back of the paws without walking |
| 4 points | Occasionally walking with sole of the paws |
| 5 points | Frequent or constant walking with sole of the paws, poor coordination; frequent or constant walking with sole of the paws, some coordination, the paws can rotate when firstly touching the ground |
| 6 points | Frequent or constant walking with sole of the paws, a certain degree of coordination, the sole of the paws is parallel to the ground when firstly touching it; walking all the time on the sole of the paws, with good coordination, the paws can rotate when firstly touching the ground |
| 7 points | Frequent or constant walking with sole of the paws, good coordination, the sole of the paws is parallel to the ground; frequent or constant walking with sole of the paws, good coordination, the sole of the paws is parallel to the ground, with severe limb balance |
| 8 points | Frequent or constant walking with sole of the paws, good coordination, the sole of the paws is parallel to the ground, with slight limb balance; frequent or constant walking with sole of the paws, good coordination, the sole of the paws is parallel to the ground, with slight limb balance, normal limb balance, and the tail is down or up and down |
| 9 points | Frequent or constant walking with sole of the paws, good coordination, the sole of the paws is parallel to the ground, with slight limb balance, the tail is always up |

The results show that the BMS score of the mice in the sham operation group is 9 points, the BMS score of the mice in the vehicle group is 6.75±1.92 points, which is significantly lower than that of the mice in the sham operation group; and the BMS score of the mice in the plasminogen group is 8.29±0.39 points, which is significantly higher than that of the mice in the vehicle group; and the statistical differences are significant (^{∗} means P<0.05, ^{∗∗∗} means P<0.001) (Fig. 8). It indicates that plasminogen may promote the recovery of motor function of the hindlimb in the mice of paraplegic model.

### Example 9: Plasminogen Promotes the Transcription of Plasminogen Gene of Spinal Cord in the Mice of Paraplegic Model

Fifteen 8-week-old C57 female mice are weighed before modeling, and all mice are randomly divided into 2 groups according to body weight; 5 mice in the blank control group, and 10 mice in the model group. The experimental mice are taken to perform pre-anesthesia, then the hairs on the back of the mice are removed. After that, the mice are completely anesthetized by respiratory anesthesia, and placed in a prone position on an anesthetic gas machine turning on 2.5% isoflurane. The skin and muscles are opened surgically, and the T9 and T10 segments are exposed to perform the laminectomy. After the spinal cord is exposed, the right half of the spinal cord between T9 and T10 is transected along the coronal plane to cause a semi-transection injury, and the wound is sutured after completion of the injury. After the mice in the blank control group are anesthetized in the same way, the skin and muscles are surgically opened to expose the T9 and T10 segments, the wound is then sutured directly ^{[6]}. The analgesic Tolfedine and penicillin potassium (200,000 units/kg; 80,000 units/ml) are administrated 24 and 72 hours before and after the operation. After the operation, all the mice are placed on a heating pad, and the neck should be kept horizontal, and the neck should not be bent until the mice are awake. Postoperative care: normally a mouse needs about 40-80ml/kg of liquid per day; after waking up from anesthesia, if the mice are found unable to eat or drink, they must be given normal saline by intraperitoneal injection to supplement body fluids, helping the mice to excrete urine until restoring the bladder's function of autonomous urination. In order to prevent the wounds from being contaminated by feces, urine and padding material on the day after the operation, a clean tissue paper should be placed on the padding material in the PC box. Three days after the completion of the surgical modeling, the postoperative mice are subjected to open-field behavioral test and scoring. According to the testing results, 10 mice in the model group are randomly divided into two groups, the vehicle group and the plasminogen group, with 5 mice in each group; the mice in the blank control group are not grouped. After the grouping is completed, all experimental mice are injected at a dose of 50 mg/kg/day through tail vein, the plasminogen group is injected with plasminogen solution (10 mg/ml), and the blank control group and vehicle group are injected with vehicle. Mice are sacrificed 6 hours after the administration, and the homogenate of spinal cord taken from the modeling part is collected for RT-PCR detection of mRNA transcribed from Plg gene, and the CT value is recorded. The primer information is shown in the Table below. The CT value of mRNA transcribed from Plg gene in 200ng total RNA is calculated.

| Target | Primer name | Primer | length |
|---|---|---|---|
| Plg | Plg-F | GACCAGTCAGATTCCTCAGTTC | 117 |
| | Plg-R | CTTCTTCCCTGTGATGGTAGTG | |

CT value: indicating the number of cycles that each PCR reaction tube undergoes when the fluorescent signal of the tube reaches a preset threshold. Studies show that there is a linear relationship between the CT value of each template and the logarithm of the initial copy number of the template. The higher the initial copy number, the smaller the CT value; vice versa.

The results show that a certain level of plasminogen mRNA exists in the spinal cord of the mice in the blank control group, and the level of plasminogen mRNA in the spinal cord of the mice in the plasminogen group is significantly higher than that of the mice in the vehicle group, and the statistical difference is close to significant (P=0.051) (Fig. 9). It indicates that plasminogen may promote the transcription of plasminogen gene in spinal cord of the mice of paraplegic model.

### Example 10: Plasminogen Promotes the Expression of Synaptophysin in Injured Spinal Cord of the Mice of Paraplegic Model

42 eight- to ten-week-old C57 female mice are weighed before modeling, and all mice are randomly divided into 2 groups according to body weight; 14 mice in the sham operation group, and 28 mice in the model group. The experimental mice are taken to perform pre-anesthesia, then the hairs on the back of the mice are removed. After that, the mice are completely anesthetized by respiratory anesthesia, and placed in a prone position on an anesthetic gas machine turning on 2.5% isoflurane. The skin and muscles are opened surgically, and the T9 and T10 segments are exposed to perform the laminectomy. After the spinal cord is exposed, the right half of the spinal cord between T9 and T10 is transected along the coronal plane to cause a semi-transection injury, and the wound is sutured after completion of the injury. After the mice in the sham operation group are anesthetized in the same way, the skin and muscles are surgically opened to expose the T9 and T10 segments, the wound is then sutured directly. The analgesic Tolfedine and penicillin potassium (200,000 units/kg; 80,000 units/ml) are administrated 24 and 72 hours before and after the operation. After the operation, all the mice are placed on a heating pad, and the neck should be kept horizontal, and the neck should not be bent until the mice are awake. Postoperative care: normally a mouse needs about 40-80ml/kg of liquid per day; after waking up from anesthesia, if the mice are found unable to eat or drink, they must be given normal saline by intraperitoneal injection to supplement body fluids, helping the mice to excrete urine until restoring the bladder's function of autonomous urination ^{[6]}. In order to prevent the wounds from being contaminated by feces, urine and padding material on the day after the operation, a clean tissue paper should be placed on the padding material in the PC box. Three days after the completion of the surgical modeling, the postoperative mice are subjected to open-field behavioral test and scoring. According to the testing results, 28 mice in the model group are randomly divided into two groups, the vehicle group and the plasminogen group, with 14 mice in each group; the mice in the sham operation group are not grouped. After the grouping is completed, the mice in the vehicle group and the sham operation group are injected with vehicle at 0.1 ml/mouse/day through tail vein, and the mice in the plasminogen group are injected with plasminogen at 1 mg/0.1 ml/mouse/day through tail vein, the first day of administration is recorded as day 1, administrating continuously for 28 days. On day 30, the mice are sacrificed, and the spinal cord of the injured part is taken out to fix in 10% formaldehyde solution. The fixed tissue samples are dehydrated with gradient ethanol and cleared with xylene before paraffin-embedding. The thickness of the tissue cross-section is 3 µm, after the sections are dewaxed and rehydrated, washing them once with water, then repairing with citric acid for 30 min, cooling at room temperature for 10 min, and rinsing gently with water; incubating with 3% hydrogen peroxide for 15 min, and circling the tissue with a PAP pen; blocking with 10% of goat serum (Vector laboratories, Inc., USA) for 1 hour, when the time is up, the goat serum is discarded. The tissue sections are incubated with rabbit-derived anti-synaptophysin antibody (Wuhan Sanying, 10842-1-AP) at 4°C overnight, then washing twice with PBS, 5 min for each time; after incubating with goat anti-rabbit IgG (HRP) antibody (Abeam) secondary antibody at room temperature for 1 hour, washing twice with PBS, 5 min for each time. The color is developed with DAB kit (Vector laboratories, Inc., USA), after washing 3 times with water, counterstaining with hematoxylin for 30 seconds, returning to blue under running water for 5 min, and then washing once with PBS. After gradient dehydration, the tissue sections are cleared and sealed, then observed under a 200-fold optical microscope.

Synaptophysin, also called synaptic vesicle protein, is a calcium-binding protein with a molecular weight of 38KDa which is located on the membrane of synaptic vesicles, and is a membrane protein closely related to synaptic structure and function. It is widely present in all nerve terminals in the body, and is specifically distributed on the membrane of presynaptic vesicles. It is involved in calcium-dependent neurotransmitter release and the circulation of synaptic vesicles, and is considered as an important symbol of synaptogenesis and synaptic remodeling ^{[8]}.

The results show that a certain level of synaptophysin (marked by arrows) is expressed in spinal cord of the mice in the sham operation group (Fig. 10A), and the level of synaptophysin in the spinal cord of modeling part of the mice in the vehicle group (Fig. 10B) is significantly lower than that of the mice in the sham operation group, and the statistical difference between the two groups is extremely significant (^{∗∗∗} means P<0.001). The level of synaptophysin in the spinal cord of the modeling part of the mice in the plasminogen group (Fig. 10C) is significantly higher than that of the mice in the vehicle group, and the statistical difference is significant (^{∗} means P<0.05) (Fig. 10D). It indicates that plasminogen may promote the expression of synaptophysin in the injured spinal cord of the mice of paraplegic model, and promote the repair of spinal cord injury.

### Example 11: Plasminogen Promotes Functional Recovery of Injured Spinal Cord Neurons in the Mice of Paraplegic Model

42 eight- to ten-week-old C57 female mice are weighed before modeling, and all mice are randomly divided into 2 groups according to body weight; 14 mice in the sham operation group, and 28 mice in the model group. The experimental mice are taken to perform pre-anesthesia, then the hairs on the back of the mice are removed. After that, the mice are completely anesthetized by respiratory anesthesia, and placed in a prone position on an anesthetic gas machine turning on 2.5% isoflurane. The skin and muscles are opened surgically, and the T9 and T10 segments are exposed to perform the laminectomy. After the spinal cord is exposed, the right half of the spinal cord between T9 and T10 is transected along the coronal plane to cause a semi-transection injury, and the wound is sutured after completion of the injury. After the mice in the sham operation group are anesthetized in the same way, the skin and muscles are surgically opened to expose the T9 and T10 segments, the wound is then sutured directly. The analgesic Tolfedine and penicillin potassium (200,000 units/kg; 80,000 units/ml) are administrated 24 and 72 hours before and after the operation. After the operation, all the mice are placed on a heating pad, and the neck should be kept horizontal, and the neck should not be bent until the mice are awake. Postoperative care: normally a mouse needs about 40-80ml/kg of liquid per day; after waking up from anesthesia, if the mice are found unable to eat or drink, they must be given normal saline by intraperitoneal injection to supplement body fluids, helping the mice to excrete urine until restoring the bladder's function of autonomous urination ^{[6]}. In order to prevent the wounds from being contaminated by feces, urine and padding material on the day after the operation, a clean tissue paper should be placed on the padding material in the PC box. Three days after the completion of the surgical modeling, the postoperative mice are subjected to open-field behavioral test and scoring. According to the testing results, 28 mice in the model group are randomly divided into two groups, the vehicle group and the plasminogen group, with 14 mice in each group; the mice in the sham operation group are not grouped. After the grouping is completed, the mice in the vehicle group and the sham operation group are injected with vehicle at 0.1 ml/mouse/day through tail vein, and the mice in the plasminogen group are injected with plasminogen at 1 mg/mouse/day through tail vein, the first day of administration is recorded as day 1, administrating continuously for 28 days. The fixed striatal tissue is dehydrated with gradient ethanol and cleared with xylene before paraffin-embedding. The thickness of the tissue section is 3 µm, and after dewaxing to water, it is stained with 0.4% tar purple staining solution (pH=3). The tissue sections are then dehydrated with gradient ethanol and cleared with xylene, then sealed with neutral gum, observed and photographed under a 400-fold optical microscope.

Nissl body, also known as chromophil substance, is a structure unique to nerve cells. It consists of many parallel rough endoplasmic reticulum and free ribosomes distributing between them, and has the function of synthesizing proteins; its number and distribution are closely related to the functional state of neurons, and are regarded as markers of neuronal activity ^{[9]}.

The results show that a certain level of Nissl bodies (marked by arrows) exists in the spinal cord of the mice in the sham operation group (Fig. 11A), and the level of Nissl bodies in the spinal cord of the modeling part of the mice in the vehicle group (Fig. 11B) is significantly higher than that of the mice in the sham operation group, the statistical difference between the two groups is extremely significant (^{∗∗∗} means P<0.001). The level of Nissl bodies in the spinal cord of the modeling part of the mice in the plasminogen group (Fig. 11C) is significantly lower than that in the vehicle group, and the statistical difference is extremely significant (^{∗∗} means P<0.01) (Fig. 11D). It indicates that plasminogen may promote the recovery of the level of Nissl bodies in the injured spinal cord of the mice of paraplegic model, and promote the repair of spinal cord injury.

### REFERENCES

[1] Suelen Adriani Marques .A simple, inexpensive and easily reproducible model of spinal cord injury in mice: Morphological and functional assessment. Journal of Neuroscience Methods 177 (2009) 183-193.
[2] Sun SW, Liang HF, Trinkaus K et al. Noninvasive detection of cuprizone induced axonal damage and demyelination in the mouse corpus callosum. Magn Reson Med. 2006 Feb;55(2):302-8.
[3] Vallieres N1, Berard JL, David S et al. Systemic injections of lipopolysaccharide accelerates myelin phagocytosis during Wallerian degeneration in the injured mouse spinal cord. Glia. 2006 Jan 1;53(1):103-13.
[4] Gotow T. Neurofilaments in health and disease. Med Electron Microsc. 2000; 33 (4):173-99.
[5] Joshi M. Development and characterization of a graded, in vivo, compressive, murine model of spinal cord injury [J]. American Journal of Mathematics, 2000, 97(2):308-311.
[6] Wen-Ge Ding,Spinal Cord Injury Causes More Damage to Fracture Healing of Later Phase than Ovariectomy in Young Mice, Connective Tissue Research, 53(2): 142-148, (2012)
[7] RV Ung, NP Lapointe , Spontaneous recovery of hindlimb movement in completely spinal cord transected mice: a comparison of assessment methods and conditions , Spinal Cord (2007) 45, 367-379.
[8] Li GL, Farooque M, Isaksson J, Olsson Y. Changes in synapses and axons demonstrated by synaptophysin immunohistochemistry following spinal cord compression trauma in the rat and mouse. Biomed Environ Sci. 2004 Sep;17(3):281-90.
[9] HEBERT AE, DASH PK. Nonredundant roles for hippocampal and entorhinal cortical plasticity in spatial memory storage [J]. Pharmacology Biochemistry and Behavior, 2004, 79(1) :143-153.

## Claims

1. A method for treating nerve injury, comprising administrating to a subject a therapeutically effective amount of one or more compounds selected from the group consisting of: a component of plasminogen activation pathway, a compound directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

2. The method according to claim 1, wherein the component of plasminogen activation pathway is selected from the group consisting of: plasminogen, recombinant human plasmin, Lys-plasminogen, Glu-plasminogen, plasmin, a variant and analog of plasminogen and plasmin comprising one or more kringle domains and protease domain of plasminogen and plasmin, mini-plasminogen, mini-plasmin, micro-plasminogen, micro-plasmin, delta-plasminogen, delta-plasmin, an activator of plasminogen, tPA and uPA.

3. The method according to claim 1, wherein the nerve injury is central nerve injury or peripheral nerve injury.

4. The method according to any one of claims 1-3, wherein the compound promotes the repair of damaged nerve.

5. The method according to any one of claims 1-4, wherein the compound promotes the regeneration of nerve myelin sheath.

6. The method according to any one of claims 1-5, wherein the compound promotes the expression of spinal neurofilament protein and the regeneration of nerve fiber.

7. The method according to any one of claims 1-6, wherein the compound promotes the recovery of sensory nerve function or the recovery of motor nerve function.

8. The method according to claim 7, wherein the compound promotes the recovery of pain sense.

9. The method according to any one of claims 1-8, wherein the compound promotes the expression of synaptophysin.

10. The method according to any one of claims 1-9, wherein the compound promotes the recovery of the level of Nissl bodies in damaged neural cells.

11. The method according to any one of claims 1-10, wherein the compound is plasminogen.

12. The method according to any one of claims 1-11, wherein the plasminogen has at least 75%, 80%, 85%, 90%, 95%, 96%, 97%, 98% or 99% sequence identity with SEQ ID NO: 2, and has the proteolytic activity of plasminogen.

13. The method according to claim 12, wherein the plasminogen is a conservative substitution variant of the plasminogen represented by SEQ ID NO: 2.

14. The method according to any one of claims 1-13, wherein the plasminogen comprises an amino acid sequence having at least 75%, 80%, 85%, 90%, 95%, 96%, 97%, 98% or 99% sequence identity with the active fragment of plasminogen represented by SEQ ID NO: 14, and has the proteolytic activity of plasminogen.

15. The method according to any one of claims 1-14, wherein the plasminogen is natural or synthetic full-length human plasminogen.
